# EUROPEAN PATENT APPLICATION

(11) **EP 1 426 067 A1**
(43) Date of publication of application: **09.06.2004**
(21) Application number: 03009176.3
(22) Date of filing: 22.04.2003
(51) Int. Cl.: A61M 1/00

(54) **No-needle blood access device for hemodialysis**

(30) Priority: 06.12.2002 JP 2002354517
(71) Applicant: Kawamura, Akio, Sapporo-shi (JP)
(72) Inventor: Kawamura, Akio, Sapporo-shi (JP)
(74) Representative: Hiltl, Elmar, Dr.

(57) **Abstract**

A no-needle blood access device for hemodialysis comprising an elongated metallic body (20) with a recess (22) being formed with a peripheral wall (24) defining a well (26) therein, a pair of shutters (40, 42) slidably housed within opposed pockets (36, 38), the shutters including through-holes (40c, 42c), a longitudinally extending through-hole (30) in the body (20), artificial conduits (12, 14) being fitted into the longitudinally extending through-hole (30), and vertical through-holes (44, 46) communicating to the respective through-holes (40c, 42c) of the shutters (40, 42) when they are opened.

## Description

### BACKGROUND OF THE INVENTION

The present invention generally relates to a no-needle blood access device for hemodialysis. More specifically, the present invention relates to a no-needle blood access device for hemodialysis which has a mechanism of simple structure and does not need a caregiver.

### DESCRIPTION OF THE PRIOR ART

Hemodialysis is used widely as a remedy for treating kidney insufficiency. In many cases, a surgical short circuit which is commonly referred to as " shunt " is implanted in a blood vessel or blood vessels of the patient suffering from serious kidney disease so as to acquire a plentiful blood flow immediately during hemodialysis, because such a patient must receive hemodialysis treatment periodically over a long period of time. Shunts are divided broadly into two categories, an internal shunt and an external shunt. The internal shunt has the drawback that needle puncture is required during hemodialysis. On the other hand, the external shunt has a high rate of thrombosis and infection, and makes daily life more inconvenient.

To overcome these drawbacks of the aforementioned shunts, a blood access device for hemodialysis given the tradename " Hemasite" (described in USP No. 4,496,350) has been developed. This Hemasite blood access device has the advantage that needle puncture is not required, but due to its complicated structure, is costly and troublesome to handle.

Mindful to the various drawbacks to prior hemodialysis, the applicant of the present invention has proposed two types of blood access devices for hemodialysis, one of which is described in USP No. 6,231,541 and the other of which is described in US Patent Application No.09/851,326 filed on May 9, 2001. The former blood access device is very useful for the patient because it does not require needle puncture, has a mechanism of simple structure, can be manufactured at a relatively low cost, and is easy to handle. The latter blood access device has an advantage that a caregiver is not required, while maintaining the above described advantages of the former device.

The above-mentioned devices proposed by the applicant of the present invention are those useful for the patient suffering from kidney disease, for which the applicant has developed a novel blood access device which handles more easily, while maintaining the advantages of those devices, by further improving upon them.

### SUMMARY OF THE INVENTION

It is therefore an object of the present invention to provide a blood access device for hemodialysis which does not require needle puncture, which has a mechanism of simple structure, which is easy to handle, which can be manufactured at a relatively low cost, and which does not need a caregiver during hemodialysis.

The above and other objects of the present invention can be accomplished by a no-needle blood access device for hemodialysis comprising, an elongated metallic body, the body being provided at its upper surface with a recess, a periphery of the recess being formed with a peripheral wall defining a well therein; a pair of shutters slidably housed within opposed pockets respectively, the pockets being formed at the upper part of the body so that each of their lower surfaces flush with the bottom surface of the recess, each of the shutters including through-holes respectively; a longitudinally extending through-hole disposed in the lower part of the body, each of first and second artificial conduits being fitted into respective ends of the longitudinally extending through-hole, the artificial conduits being anastomosed to a targeted artery or vein; and a pair of vertical through-holes disposed at portions of the body each communicating to the respective through-holes of the shutters when they are opened; whereby the device is arranged such that, when each of the shutters is slided in a direction away from each other, the well is in communication with each of the artificial conduits through the logitudinally extending through-hole and the vertical through-holes of the body and each of the through-holes of the shutters, and when each of the shutters is slided in a direction near to each other, the well is out of communication with each of the artificial conduits.

In a preferred aspect of the present invention, each of the shutters includes a horizontal portion housed within the pocket and a vertical portion formed in the end facing with respect to each other respectively, each of the through-holes of the shutters being provided at the vertical portion.

In another preferred aspect of the present invention, each of the vertical portions is provided with a recess which is used when the shutters are opend and closed.

In a further preferred aspect of the present invention, the device further comprises a cannula assembly connected to a dialyzer, the cannula assembly including a pair of cannulas, one end of each of the cannulas being provided with an adapter for mounting the cannula to the body, the adapter being provided with a locking member for preventing the cannula from being removed.

In a further preferred aspect of the present invention, the locking member includes a projection for locking in a groove formed in a side surface of the vertical portion of each of the shutters.

In a further preferred aspect of the present invention, the locking member includes a groove for being locked in a projection formed in a side surface of the vertical portion of each of the shutters.

The above and other objects and features of the present invention will become apparent from the following description made with reference to the accompanying drawings.

### BRIEF DESCRIPTION OF THE DRAWINGS

Figure 1 is a diagrammatic view of a no-needle blood access device for hemodialysis of a preferred embodiment of the present invention which is implanted in a human body.
Figure 2 is a perspective view of the no-needle blood access device for hemodialysis of Figure 1.
Figure 3 is a cross sectional view of the device of Figure 1 showing the condition wherein the well is out of communication with each of the artificial conduits.
Figure 4 is a cross sectional view of the device of Figure 1 showing the condition wherein the well is in communication with each of the artificial conduits.
Figure 5 is an enlarged view showing the circumference of the shutters of the no-needle blood access device for hemodialysis of Figure 1.
Figure 6 is an enlarged view showing the condition wherein the cannula is inserted into the through-hole of the shutter of the no-needle blood access device for hemodialysis of Figure 1.
Figure 7 is an enlarged view showing the alternative condition wherein the cannula is inserted into the through-hole of the shutter of the no-needle blood access device for hemodialysis of Figure 1.
Figure 8 is a diagrammatic view showing the condition wherein the no-needle blood access device for hemodialysis of the present invention is used to hemodialyze.
Figure 9 is a view similar to Figure 3 showing an alternative form of a no-needle blood access device for hemodialysis of the present invention.

### DESCRIPTION OF THE PREFERRED EMBODIMENTS

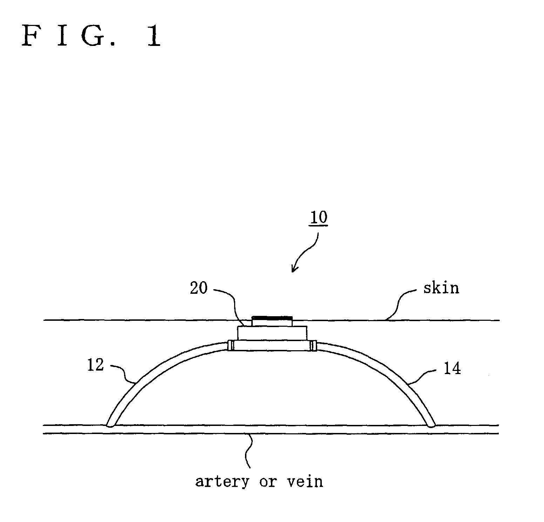

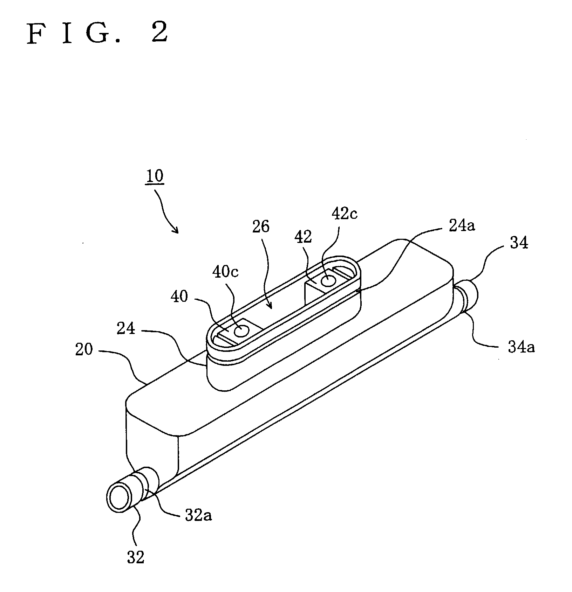

The preferred embodiments of the present invention will now be explained with reference to the accompanied drawings. A no-needle blood access device for hemodialysis generally indicated by a reference numeral 10 in Figures 1 and 2 which is an embodiment of the present invention comprises an elongated metallic body 20 having a generally rectangular cross section. The body 20 is provided at its upper surface with a longitudinally extending recess 22. The periphery of the recess 22 of the body 20 is formed with a generally vertically and upwardly extending peripheral wall 24 and the peripheral wall 24 defines a well 26 therein.

Preferably, the body 20 is made from titanium which is light and biocompatible, and the part of the body 20 with which blood comes into contact is covered with pyrolitic carbon which is an anticoagulant substance. Further, preferably, the external surface of each of the body 20 and the peripheral wall 24 is covered with a biocom patible fiber 28 such as dacron velour in order to enhance the fusion with human tissue and provide a barrier to bacteria invading from the outside.

The body 20 is provided at its lower part with a longitudinally extending through-hole 30. A pipe member 32 is mounted on the position at which one end of the through-hole 30 locates, and a pipe member 34 is mounted on the position at which the other end of the through-hole 30 locates. Each of artificial conduits 12 and 14 is fitted into the respective pipe members 32 and 34. Serration 32a and 34a is provided at the external surface of each of the pipe members 32 and 34 to prevent the artificial conduits 12 and 14 fitted into the pipe members 32 and 34 from being removed, respectively.

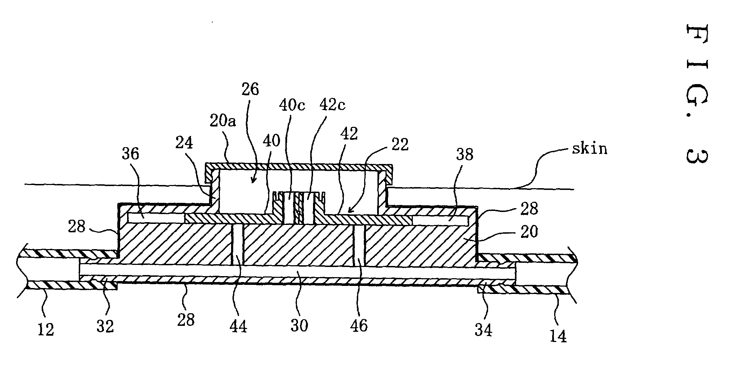

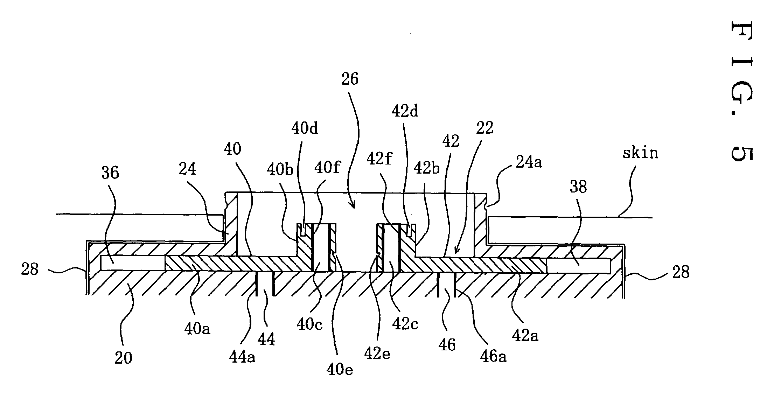

As shown in Figures 3 to 5, the body 20 is provided at its upper part with longitudinally extending opposed pockets 36 and 38 so that each of their lower surfaces are flush with the bottom surface of the recess 22, and each of a pair of shutters 40 and 42 is slidably housed within the pockets 36 and 38 respectively. As best shown in Figure 5, the shutter 40 includes a horizontal plate portion 40a and a vertical portion 40b formed in the end facing to the shutter 42. The shutter 42 also includes a horizontal plate portion 42a and a vertical portion 42b formed in the end facing to the shutter 40. Each of the vertical portions 40b and 42b is provided with vertically extending through-holes 40c and 42c respectively.

Preferably, each of the vertical portions 40b and 42b is formed at its upper surface with recesses 40d and 42d for inserting a tool (not shown) to open and close the shutters 40 and 42 during their sliding movement respectively. Further, preferably, each of the vertical portion 40b and 42b is provided at its side surface facing with respect to each other with grooves 40e and 42e for preventing cannulas 52, which are described later, from being removed. Furthermore, preferably, each of the through-holes 40c and 42c is provided at its internal surface with oil seals 40f and 42f respectively.

The body 20 is provided with vertical through-holes 44 and 46 at portions each communicating to the respective through-holes 40d and 42d of the shutters 40 and 42 when they are opened (in other words, when they are slided in a direction away from each other). Thereby, the well 26 is in communication with each of the artificial conduits 12 and 14 through the through-holes 40d and 42d of the shutters 40 and 42 and the through-holes 44 and 46 of the body 20. Further, preferably, each of the through-holes 44 and 46 is provided at their internal surface with oil seal 44a and 46a respectively.

As shown in Figure 3, the top of the body 20 is covered with a cap 20a when the blood access device 10 is not in use. Further, preferably, the external surface of the peripheral wall 24 is formed with a serration 24a to prevent the cap 20a from being removed.

The blood access device 10 further comprises a cannula assembly 50 connected to a dialyzer. The cannula assembly 50 includes a pair of cannulas 52, and one end of each of the cannulas 52 is provided with an adapter 54 for mounting the cannula assembly 50 to the body 20. Each of the cannulas 52 is made of a conventional flexible material.

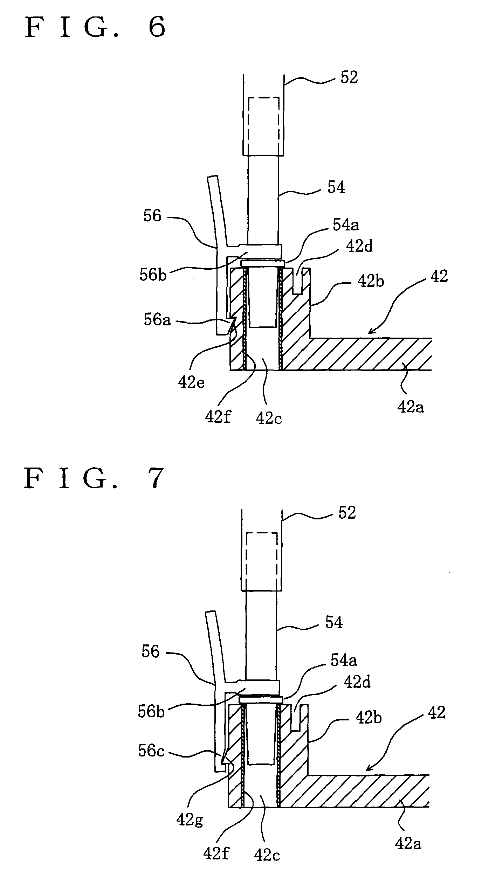

As shown in Figure 6, the adapter 54 is made of tube of synthetic resin material such as plastic, etc. and preferably, the diameter of the leading end of the adapter 54 is selected to be slightly smaller than the diameter of each of the through-holes 40d and 42d and the leading end of the adapter 54 is shaped to be slightly convergent to facilitate the insertion of the adapter 54 into each of the through-holes 40d and 42d of the shutters 22 during hemodialysis. Further, the adapter 54 is provided with a stopper 54a for preventing it from being inserted into each of the through-holes 40d and 42d excessively. Furthermore, the adapter 54 is also provided with a locking member 56 for preventing the cannula 52 from being removed during hemodialysis. A leading end of the locking member 56 is provided with a projection 56a for locking in a groove 42e formed in the side surface of the vertical portion 42b of the shutter 42, and the locking member 56 itself is mounted on the adapter 54 by a ring 56a. The shutter 40 is also provided at the side surface of the vertical portion 40b with a groove 40e, as shown in Figure 5.

Alternatively, as shown in Figure 7, the side surface of the vertical portion 42b of the shutter 42 may be provided with a projection 42g, and the leading end of the locking member 56 may be provided with a groove 56c for being locked in the projection 42g.

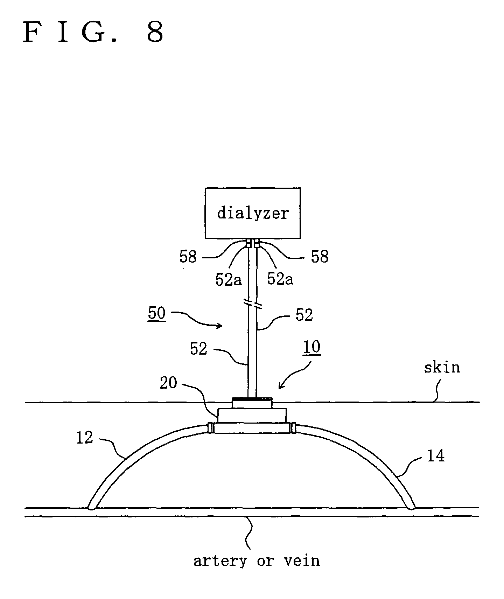

Each of the cannulas 52 is a tube adapted to define a connecting circuit leading to the dialyzer, and each of them has an internal diameter approximately equal to the diameter of each of the through-holes 40d, 42d, 44 and 46. As shown in Figure 8, each of the cannula 52 is provided at the end proximal to the dialyzer with a terminal 52a for connecting to a terminal 58 of the dialyzer. The terminal 52a may be a conventional screw type terminal. Each of the cannulas 52 has a length (at least 3 meters) sufficient for the patient to move around with relative freedom during hemodialysis.

The cannulas 52 may consist of either two separate tubes or by combining two tubes so they appear as one tube.

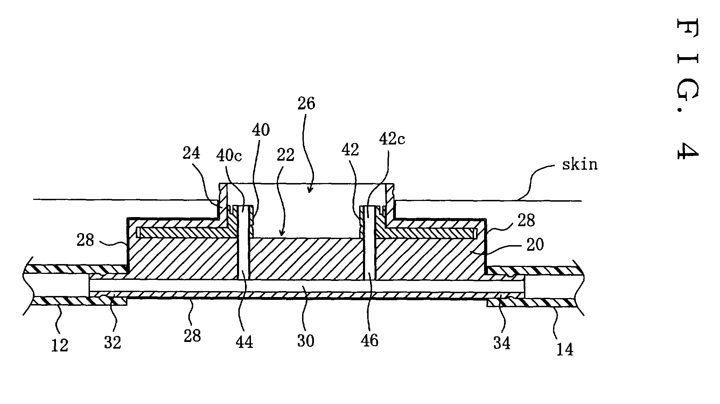

The thus constituted no-needle blood access device for hemodialysis 10 operates as follows. Firstly, as shown in Figure 1 , the device 10 is implanted in a desired area of the upper arm, etc. of the patient and each of the artificial conduits 12 and 14 is anastomosed to the targeted artery or vein. When it is to be hemodialyzed, the cap 20a is removed from the body 20, and the leading end (therefore, the adapter 54) of each of the cannulas 52 of the cannula assembly 50, which is connected to the dialyzer, is inserted into the through-holes 40d and 42d of the shutters 40 and 42 of the body 20 respectively. Neither of the cannulas 52 is to be removed from the through-hole by the locking member 56. Thereafter, each of the shutters 40 and 42 is slided in a direction away from each other by a tool such as a pincette to bring the through-hole 40c in communication with the through-hole 44 and bring the through-hole 42c in communication with the through-hole 46, as shown in Figure 4, thereby effecting hemodialysis. When hemodialysis is completed, each of the shutters 40 and 42 is slided in a direction near to each other to bring the through-hole 40c out of communication with the through-hole 44 and bring the through-hole 42c out of communication with the through-hole 46. Then, each of the cannulas 52 is withdrawn, and the body 20 is covered with the cap 20a, as shown in Figure 3.

The present invention has thus been shown and described with reference to specific embodiments. However, it should be noted that the present invention is in no way limited to the details of the described arrangements but changes and modifications may be made without departing from the scope of the appended claims.

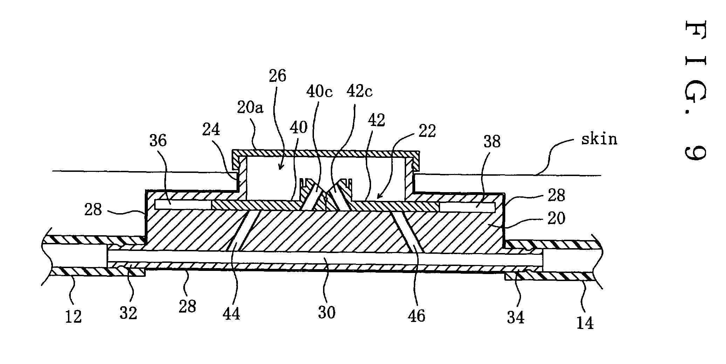

For example, although each of the through-holes 40c and 42c of the shutters 40 and 42 and each of the through-holes 44 and 46 of the body 20 are formed to extend in the vertical direction in the above described embodiments, they may be formed to extend in the inclined direction, as shown in Figure 9.

Furthermore, although each of the artificial conduits 12 and 14 is anastomosed to the same artery or vein in the above described embodiments, each of them may be anastomosed to separate arteries or veins.

According to the present invention, since the artery or vein can be in communication with the dialyzer so that the leakage of blood does not occur during hemodialysis, hemodialysis can be effected without a caregiver. Further, since the body has a simple structure, a blood access device for hemodialysis is provided which does not require needle puncture, can be manufactured at a relatively low cost and is easy to handle.

## Claims

1. A no-needle blood access device for hemodialysis, comprising
an elongated metallic body (20), the body (20) being provided at its upper surface with a recess (22), a periphery of the recess (22) being formed with a peripheral wall (24) defining a well (26) therein;
a pair of shutters (40, 42) slidably housed within opposed pockets (36, 38) respectively, the pockets (36, 38) being formed at the upper part of the body (20) so that each of their lower surfaces flush with the bottom surface of the recess (22), each of the shutters (40, 42) including through-holes (40c, 42c) respectively;
a longitudinally extending through-hole (30) disposed in the lower part of the body (20), each of first and second artificial conduits (12, 14) being fitted into respective ends (32, 34) of the longitudinally extending through-hole (30), the artificial conduits (12, 14) being anastomosed to a targeted artery or vein; and
a pair of vertical through-holes (44, 46) disposed at portions of the body (20) each communicating to the respective through-holes (40c, 42c) of the shutters (40, 42) when they are opened;
whereby the device is arranged such that, when each of the shutters (40, 42) is slided in a direction away from each other, the well (26) is in communication with each of the artificial conduits (12, 14) through the longitudinally extending through-hole (30) and the vertical through-holes (44, 46) of the body (20) and each of the through-holes (40c, 42c) of the shutters (40, 42), and when each of the shutters (40, 42) is slided in a direction near to each other, the well (26) is out of communication with each of the artificial conduits (12, 14).

2. A device according to claim 1, wherein each of the shutters (40, 42) including a horizontal portion (40a, 42a) housed within the pocket (36, 38) and a vertical portion (40b, 42b) formed in the end facing with respect to each other respectively, each of the through-holes (40c, 42c) of the shutters (40, 42) being provided at the vertical portion (40b, 42b).

3. A device according to claim 1 or 2, wherein each of the vertical portions (40b, 42b) is provided with a recess (40d, 42d) which is used when the shutters (40, 42) are opened and closed.

4. A device according to any of claims 1 to 3, further comprising a cannula assembly (50) connected to a dialyzer, the cannula assembly (50) including a pair of cannulas (52), one end of each of the cannulas (52) being provided with an adapter (54) for mounting the cannula (52) to the body (20), the adapter (54) being provided with a locking member (56) for preventing the cannula (52) from being removed.

5. A device according to claim 4, wherein the locking member (56) including a projection (56a) for locking in a groove (42e) formed in a side surface of the vertical portion (40b, 42b) of each of the shutters (40, 42).

6. A device according to claim 4, wherein the locking member (56) including a groove (56c) for being locked in a projection (42g) formed in a side surface of the vertical portion (40b, 42b) of each of the shutters (40, 42).
